(19) Europäisches Patentamt
European Patent Office
Office européen des brevets

(11) EP 3 779 812 A1

(12) EUROPEAN PATENT APPLICATION
published in accordance with Art. 153(4) EPC

(43) Date of publication:
17.02.2021 Bulletin 2021/07

(51) Int Cl.:
$G06Q\ 10/04$ (2012.01)

(21) Application number: 18911933.2

(22) Date of filing: 29.11.2018

(86) International application number:
PCT/JP2018/043910

(87) International publication number:
WO 2019/187341 (03.10.2019 Gazette 2019/40)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 30.03.2018 JP 2018067622

(71) Applicant: NEC Solution Innovators, Ltd.
Koto-ku, Tokyo 136-8627 (JP)

(72) Inventors:
• TAMANO Hiroshi
Tokyo 136-8627 (JP)
• TANIGUCHI Atsushi
Tokyo 136-8627 (JP)
• SEIKE Katsuyo
Tokyo 136-8627 (JP)
• TANAKA Hirofumi
Tokyo 136-8627 (JP)
• TAJIRI Toshikazu
Tokyo 136-8627 (JP)

(74) Representative: Vossius & Partner
Patentanwälte Rechtsanwälte mbB
Siebertstrasse 3
81675 München (DE)

(54) INDEX COMPUTATION DEVICE, PREDICTION SYSTEM, PROGRESS PREDICTION EVALUATION METHOD, AND PROGRAM

(57) To automatically determine the invalidity of series data including the predicted value obtained based on assumed conditions. The index computation device 60 includes invalidity score output means 61 that outputs, when series data of a predetermined prediction target item is input, the series data including three or more pieces of data which indicate the value of the prediction target item in association with time, and at least one of which is data indicating a predicted value, an invalidity score that is an index indicating the invalidity of the series data and is based on an error between a curve model obtained by fitting the series data to a predetermined function form and the series data.

FIG. 11

INDEX COMPUTATION DEVICE 60

INVALIDITY SCORE OUTPUT MEANS 61

**Description**

Technical Field

The present invention relates to an index computation device for determining the validity of series data in a transition graph or the like whose value changes with the passage of time, and a prediction system using the index computation device. The present invention also relates to a progress prediction evaluation method and a progress prediction evaluation program for evaluating the validity of progress prediction including predicted values at two or more time points of a variable whose value changes with the passage of time.

Background Art

For example, let us consider predicting a secular change of an inspection value of an employee or the like measured in a health checkup or the like, a disease onset probability of a lifestyle-related disease based on it, or the like, and giving advice to each employee regarding health. Specifically, let us consider a case where future state (secular change of an inspection value, a disease onset probability, etc.) when the current lifestyle habits continue for three years is predicted based on past health checkup results and data showing lifestyle habits at that time, and then, an industrial physician, an insurer, etc. propose (health-instruct) review of the lifestyle habits, etc., or the employee himself/herself self-checks it.

In that case, the following method can be considered as a method for obtaining the transition of the predicted value. First, learn a prediction model that obtains a predicted value one year ahead from past data. For example, learn a prediction model that in association with past actual values (inspection values) of a prediction target, uses training data indicating further past inspection values that can be correlated with the past actual values, the attributes (age, etc.) of the prediction target person, and lifestyle habits at that time, and then, uses a prediction target item after 1 year as a target variable and other items that can be correlated with it as explanatory variables. Then, with respect to the obtained prediction model, the process of inputting the explanatory variables and obtaining a predicted value one year ahead while changing a time point (prediction time point) at which the value to be predicted is obtained is repeated for several years. At this time, by keeping the items related to lifestyle habits among the explanatory variables constant, it is possible to obtain the transition of the predicted value when the current lifestyle habits are continued for three years.

If health guidance or self-check is performed based on such transition of the predicted value, more effective and efficient disorder prevention/health promotion and behavior change for that purpose of a person being instructed or himself/herself are expected.

As a technology related to health prediction and health support, NPL 1 describes an example of a health risk appraisal system that predicts a health risk based on the results of health checkup and lifestyle. The health risk appraisal system described in NPL 1 includes two subsystems, an inspection value prediction system and an onset prediction system. The inspection value prediction system indicates the degree of improvement in inspection results associated with lifestyle improvement, for people who are currently having mild inspection abnormalities and have a lifestyle problem. In addition, the onset prediction system predicts the disease incidence rate for people who do not have inspection abnormalities at present but have a biased lifestyle, when they continue to have an undesirable lifestyle and when they improve their lifestyle. According to NPL 1, health support by any of the subsystems is also interactively performed between a medical staff and a patient, and the patient can recognize the effect of his/her behavior change.

However, when the future transition of the predicted value under such an assumption is simply obtained by using a prediction model, the obtained transition of the predicted value may show a change different from general findings.

Regarding verification of the predicted value, for example, PTL 1 describes a method for evaluating a demand prediction model. The method described in PTL 1 incorporates the demand actual value and the demand predicted value in the evaluation period of the demand prediction model, calculates the deviation value between the demand actual value and the demand predicted value for each supply cycle of the product, and evaluates the demand prediction model based on the calculated deviation value.

Citation List

Patent Literature

PTL 1: Japanese Patent Application Laid-Open No. 10-228463

Non Patent Literature

NPL 1: Hidetaka Takahashi, Katsumi Yoshida, "HRA (Health Risk Appraisal System) for Lifestyle Improvement", Journal of Japan Society of Human Dock (JHD) Vol.11 No.4 1997, p.123-128.

Summary of Invention

Technical Problem

However, the prediction system described in NPL 1 does not consider the validity of the transition of the predicted value at all.

In addition, the method described in PTL 1 performs comprehensive evaluation of past predicted values using actual values, and assumes that at least actual values at the time of prediction are obtained. However, in the progress prediction that predicts values at multiple future time points using the above assumptions, not all the conditions assumed at that time point may match. That is, it is not always possible to obtain actual values that match all the conditions at the time of prediction. Further, the method of PTL 1 that performs determination after obtaining actual values cannot be applied to such applications that automatically determine the validity of the prediction result before displaying it.

It should be noted that it may be possible to search for past actual values of other people having similar attributes under the same conditions and substitute them for the actual values. However, for all the subjects, it is not always possible to find values, at all prediction time points, that match the values of many explanatory variables including the past inspection values and the assumed conditions. In the above-mentioned progress prediction, which predicts inspection values that originally tend to cause individual differences, even if the validity of future transition of predicted values under tentative conditions is determined based on a very small number of individual actual values, it is difficult to obtain accurate determination results.

The problem is that there is no objective index that can determine the validity of the transition of the predicted value obtained based on the assumed conditions, and it is difficult to determine whether or not the prediction result is valid, and if it is not, how invalid it is. Note that "invalid" here means uncertain (unexplainable) at least in the knowledge of the person who handles the predicted value (in the above example, an industrial doctor who performs health instruction, an insurer, etc., the person who performs self-check, etc.).

For example, let us consider giving advice based on the transition of the predicted value obtained by predicting the disease onset probability of a lifestyle-related disease and the inspection values related to it when the explanatory variables related to lifestyle habits are constant.

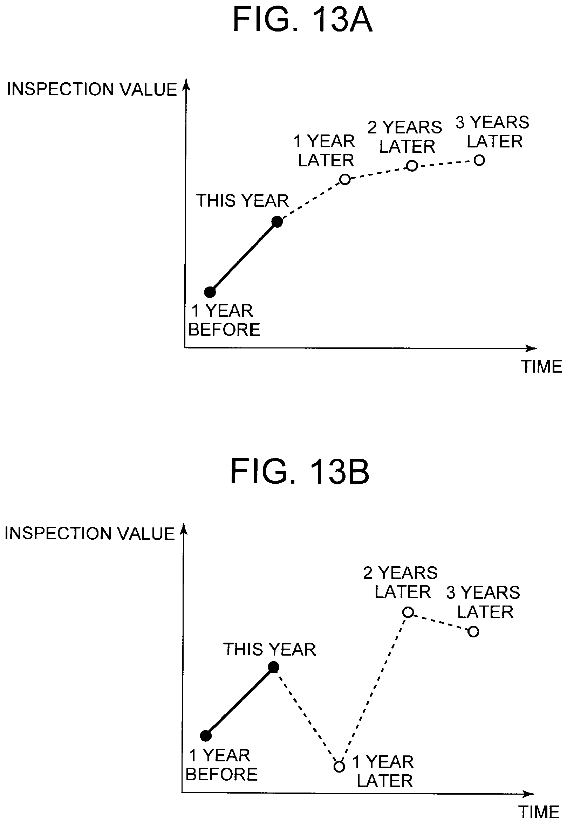

According to the general feeling, if the lifestyle habits are kept constant, for example, as shown in Fig. 13A, it is natural that the disease onset probability of a lifestyle-related disease and the transition of inspection values related thereto gradually approach some value.

However, if the progress prediction is performed simply by repeatedly applying the prediction model that predicts the predicted value at the next time point (for example, one year later) in a predetermined prediction time unit, although the lifestyle habits are kept constant, as shown in Fig. 13B, there is a possibility that the prediction result will be shown in the form of a bumpy graph that rises and falls with the direction of change (plus or minus) not fixed, such that it changes upwards in one year and downwards in another year. There is a problem that even if an advice is given to improve lifestyle habits based on such a prediction result, since both the person giving the advice and the person receiving the advice feel uncomfortable, the prediction result cannot be used as a basis for the advice.

For example, like the above health simulation, when it is considered that an industrial physician, an insurer, etc. propose (health-instruct) review of the lifestyle habits based on the results of predicting the progress based on past health checkup results and data showing lifestyle habits at that time, or the employee himself/herself self-checks it, it is important for a prediction mechanism how to prevent the above-described prediction result that cannot be interpreted from being output.

To that end, when having obtained series data including a predicted value obtained based on the assumed conditions, it is desirable to obtain an objective index representing whether the transition of the predicted value indicated by the obtained series data is valid, or if it is not, how invalid it is, without waiting for the actual value.

For example, the method of visually determining the output of the prediction result by a domain expert has a problem that it not only requires a high cost but also takes time to output. In addition, even when selecting a model that cannot be interpreted as described above from among several prediction model candidates, there is a similar problem in the method of visually determining the output by a domain expert.

In view of the problems described above, the present invention provides an index computation device, a prediction system, a progress prediction evaluation method, and a progress prediction evaluation program that can automatically determine the invalidity of series data including a predicted value obtained based on the assumed conditions.

Solution to Problem

An index computation device according to the present invention includes invalidity score output means that outputs, when series data of a predetermined prediction target item is input, the series data including three or more pieces of data which indicate the value of the prediction target item in association with time, and at least one of which is data indicating a predicted value, an invalidity score that is an index indicating the invalidity of the series data and is based on an error between a curve model obtained by fitting the series data to a predetermined function form and the series data.

In addition, a prediction system according to the present invention includes prediction means that obtains a predicted value at a predetermined prediction time point by using a learned prediction model for a predetermined prediction target item, and generates series data including the obtained predicted value and including three or more pieces of data which indicate the value of the prediction target item in association with time, and at least one of which is data indicating a predicted value, invalidity score calculation means that calculates an invalidity score that is an index indicating the invalidity of the series data and is based on an error between a curve model obtained by fitting the series data to a predetermined function form and the series data, and evaluation means that evaluates the series data, a predicted value included in the series data, or a prediction model that has obtained the predicted value, based on the invalidity score.

Further, a progress prediction evaluation method according to the present invention includes, when series data of a predetermined prediction target item is input, the series data including three or more pieces of data which indicate the value of the prediction target item in association with time, and at least one of which is data indicating a predicted value, calculating, by an information processing device, an invalidity score that is an index indicating the invalidity of the series data and is based on an error between a curve model obtained by fitting the series data to a predetermined function form and the series data, and evaluating, by the information processing device, series data, a predicted value included in the series data, or a prediction model that has obtained the predicted value, based on the invalidity score.

Further, a progress prediction evaluation program according to the present invention causes a computer to execute the processes of, when series data of a predetermined prediction target item is input, the series data including three or more pieces of data which indicate the value of the prediction target item in association with time, and at least one of which is data indicating a predicted value, calculating an invalidity score that is an index indicating the invalidity of the series data and is based on an error between a curve model obtained by fitting the series data to a predetermined function form and the series data, and evaluating series data, a predicted value included in the series data, or a prediction model that has obtained the predicted value, based on the invalidity score.

Advantageous Effects of Invention

According to the present invention, it is possible to automatically determine the invalidity of series data including a predicted value obtained based on assumed conditions.

Brief Description of Drawings

[Fig. 1] It depicts a block diagram showing a configuration example of an index computation device of a first exemplary embodiment.

[Fig. 2] It depicts an explanatory diagram showing an outline of an invalidity score.

[Fig. 3] It depicts an explanatory diagram showing an example of a graph display of series data.

[Fig. 4] It depicts an explanatory diagram showing an outline of a method of calculating x_scale.

[Fig. 5] It depicts a flowchart showing an example of an operation of the index computation device of the first exemplary embodiment.

[Fig. 6] It depicts a block diagram showing a configuration example of a prediction system according to a second exemplary embodiment.

[Fig. 7] It depicts a flowchart showing an example of an operation of the prediction system of the second exemplary embodiment.

[Fig. 8] It depicts a block diagram showing a configuration example of a prediction system according to a third exemplary embodiment.

[Fig. 9] It depicts a flowchart showing an operation example of the prediction system of the third exemplary embodiment.

[Fig. 10] It depicts a schematic block diagram showing a configuration example of a computer according to an exemplary embodiment of the present invention.

[Fig. 11] It depicts a block diagram showing an outline of an index computation device of the present invention.

[Fig. 12] It depicts a block diagram showing an outline of a prediction system of the present invention.

[Fig. 13] It depicts a graph showing an example of transition of predicted values.

Description of Embodiments

First exemplary embodiment

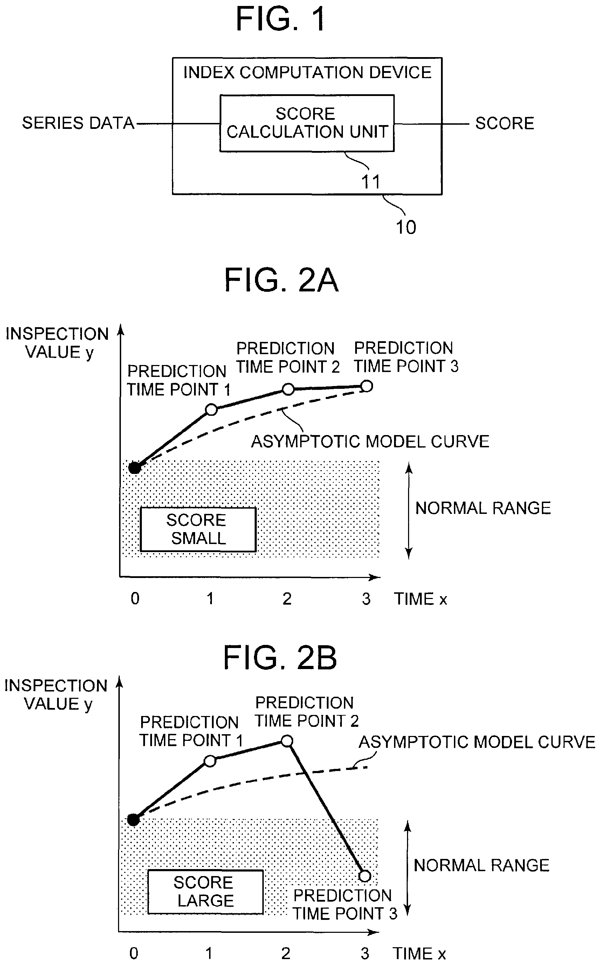

An exemplary embodiment of the present invention will be described below with reference to drawings. Fig. 1 is a block diagram showing a configuration example of the index computation device of the present exemplary embodiment. An index computation device 10 shown in Fig. 1 includes a score calculation unit 11.

The score calculation unit 11 receives, as an input, series data including three or more pieces of data which indicate the value of a prediction target item in association with time, and at least one of which indicates a predicted value, and calculates and outputs an invalidity score that is an index indicating invalidity of the series data.

In the following, the invalidity score is calculated as an index indicating how far the input series data is from the predetermined asymptotic model.

As described above, it is natural that the predicted value of the inspection value approaches a certain value when certain lifestyle habits are continued. Therefore, the score calculation unit 11 calculates an unexplainable degree (invalidity score) of the input predicted value sequence based on the error between the input series data and a predetermined asymptotic model (see Fig. 2). Note that Fig. 2 is an explanatory diagram showing an outline of the invalidity score, Fig. 2A is an example when the invalidity cocoa is small, and Fig. 2B is an example when the invalidity cocoa is large. The shaded area in Fig. 2 represents the normal range of the inspection value.

Here, the asymptotic model is a curve model that represents a curve having an asymptote parallel to the X-axis when time is the X-axis and the prediction item is the Y-axis, and more specifically, a curve model expressed by a function in which when $x \to \infty$, $y(x)$ converges to a certain value. Here, x represents a point (coordinate) on the time axis corresponding to each data in the series data, and y(x) represents a prediction item value at the time point x. The asymptotic model may be a curve model represented by a function that satisfies at least the condition represented by the following formula (1). Here, a is an arbitrary constant. The existing asymptote is not limited to one, and includes, for example, the one represented by a function in which two asymptotes exist such as a function called a logistic function or an arctangent function.

[Math 1]

$$\lim_{x \to \infty} y(x) = a \quad \cdot \ \cdot \ \cdot \quad (1)$$

The score calculation unit 11, as the asymptotic model, can use a curve model represented by one predetermined function form, but may use a model obtained, for example, by fitting the input series data to a predetermined two or more function forms that satisfy the above condition.

The fitting may be performed, for example, by searching for a solution ($\theta$ with a hat) of a model parameter $\theta$ that minimizes a predetermined loss function as shown in the following formula (2).

[Math 2]

$$\hat{\theta} = \text{argmin}_\theta \sum_n loss(f(x_n, \theta), y_n) \quad \cdot \ \cdot \ \cdot \quad (2)$$

$$ex)$$

$$loss(y1, y2) = (y1 - y2)^2,$$

$$f(x, \theta) = c + ba^x$$

$$s.t. \ \theta = \{a, b, c\}, \ 0 < a < 1$$

In formula (2), n represents a time point of the value at which fitting is performed, loss() represents an error function, and f() represents a function form of the fitting destination. It should be noted that $f(x_n, \theta)$ represents the output when an arbitrary time point $x_n$ and a set of model parameters $\theta$ is given to the function form f(), and $f(x_n, {}^\wedge\theta)$ represents the output at an arbitrary time point $x_n$ in the asymptotic model obtained by fitting. In the example shown in formula (2), the square loss is used as the error function, but the error function is not limited to the square loss.

The score calculation unit 11 may calculate, for example, an error between the asymptotic model thus obtained and the series data of the input predicted value, and output it as an invalidity score.

The score calculation unit 11 may output, for example, an error value (error) represented by the following formula (3) as an invalidity score.
[Math 3]

$$error = \sum_n loss\big(f(x_n, \hat{\theta}), y_n\big) \quad \cdot \cdot \cdot \quad (3)$$

The score calculation unit 11 may specify the data used for fitting and the data used for calculating the error independently. The score calculation unit 11 can also accept these designations from the user. At this time, the data used for the fitting (the data belonging to the first group) and the data used for calculating the error (the data belonging to the second group) do not have to completely match.

As an example, when the series data including N piece of data is input, the fitting is performed using the first half N'(where N' < N) piece of data, and the error calculation may be performed using the value of remaining pieces of data (N-N' pieces) or all pieces of data (N pieces). In addition, as another example, it is also possible to perform fitting using data at the time points that are not continuous in the series data such as the first, third, and fifth data, and perform error calculation using all pieces of data.

For example, when series data including five pieces of data is input, the score calculation unit 11 may perform fitting and error calculation as follows, for example.

[Example of pattern for fitting and error calculation]

- The fitting is performed with the first three pieces of data, and the error calculation is performed with the second two pieces of data.
- The fitting is performed with the first three pieces of data, and the error calculation is performed with all pieces of data.
- The fitting is performed with the first, third, and fifth data, and the error calculation is performed with all pieces of data.

Here, the number of pieces of data (the number of predicted values) Np included in the series data input to the score calculation unit 11 is not particularly limited, but at least one is included. In practice, it is preferable that the series data include at least data indicating predicted values for the number of time points at which the progress prediction was performed. Note that the series data may include data indicating past actual values, and in that case, the above N represents the total number of pieces of data including data indicating past actual values. Note that N is presumed to be three or more, but from the viewpoint of fitting accuracy, for example, four or more is more preferable.

In addition, when the series data includes the data indicating the actual value, the error calculation may be performed using only the data indicating the predicted value.

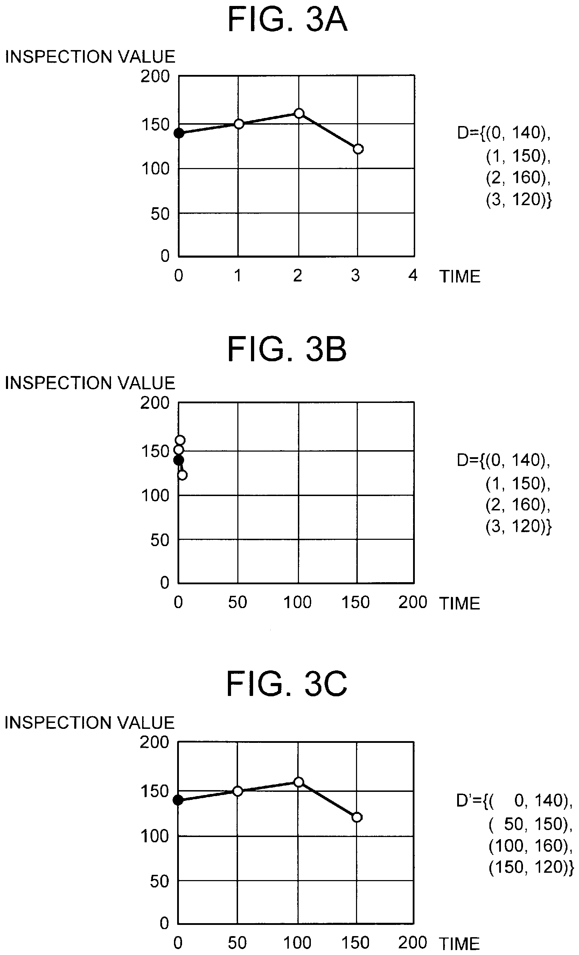

Further, the score calculation unit 11 may rescale the x-coordinate (the value representing the time corresponding to the value of each prediction item), which is the value of the X-axis, before performing the fitting. As shown in Fig. 3A, when displaying the series data in a graph, it is conceivable that the ordinate and the abscissa have greatly different numerical units (scale width), such that the unit of the scale of the vertical axis (Y axis: predicted value) is 50, the unit of the scale of the horizontal axis (X axis: time) is 1, etc. In that case, even if the fitting is performed using the numerical values as they are, the asymptotic model expected by the viewer cannot be obtained. This is because, as shown in Fig. 3B, the fitting is performed in the graph shape when the values that are greatly different in scale on the X-axis and the Y-axis are displayed at equal intervals in a graph, so when actually displayed, fitting is performed to a curve having a shape different from that of a curve that is a valid graph shape.

In order to eliminate such inconvenience, as shown in Fig. 3B, it is preferable to perform the fitting after rescaling the value of time (x coordinate) associated with each data included in the series data according to the actual display.

For example, the score calculation unit 11, when the parameter (display parameter) relating to the display such as the scale setting of the graph displaying the input series data is obtained, may convert the x coordinate so that the width of the main scale of the X axis has the same unit (50 units in the example of Fig. 3C) as the width of the main scale of the Y axis. In this case, whereas the unit of the width of the X axis main scale is 1, the unit of the width of the Y axis main scale is 50 (50 times), so the x coordinate is also 50 times. Hereinafter, such a scaling factor for rescaling the x axis may be referred to as a rescale parameter x_scale.

The score calculation unit 11 can also obtain the rescale parameter x_scale as follows. The score calculation unit 11 can also input the display parameter which is a parameter when displaying the series data together with the series data, and calculate the rescale parameter xscale based on the information obtained from the series data and the display parameter.

The following formula (4) is an example of a formula for calculating the rescale parameter x_scale. In formula (4), $y_{max}$ and $y_{mim}$ represent the maximum and minimum values of the prediction items included in the series data, respectively. $N_d$ represents the number of pieces of data (the score of the prediction item to be displayed) included in the series data. Further, Ar represents the aspect ratio Ar (that is, the ratio of the horizontal width to the vertical width) of the display graph of the series data. In formula (4), 0.8 represents the display ratio in the vertical direction and 0.9 represents the display ratio in the horizontal direction, but these values are appropriately adjusted.

$$x\_scale = ((y_{max} - y_{min}) / 0.8*Ar*0.9) / (N_d-1))$$

$$... \quad (4)$$

In the example shown in the formula (4), $y_{max}$, $y_{mim}$, and $N_d$ correspond to the information obtained from the series data, and Ar, the vertical display ratio, and the horizontal display ratio correspond to the display parameters.

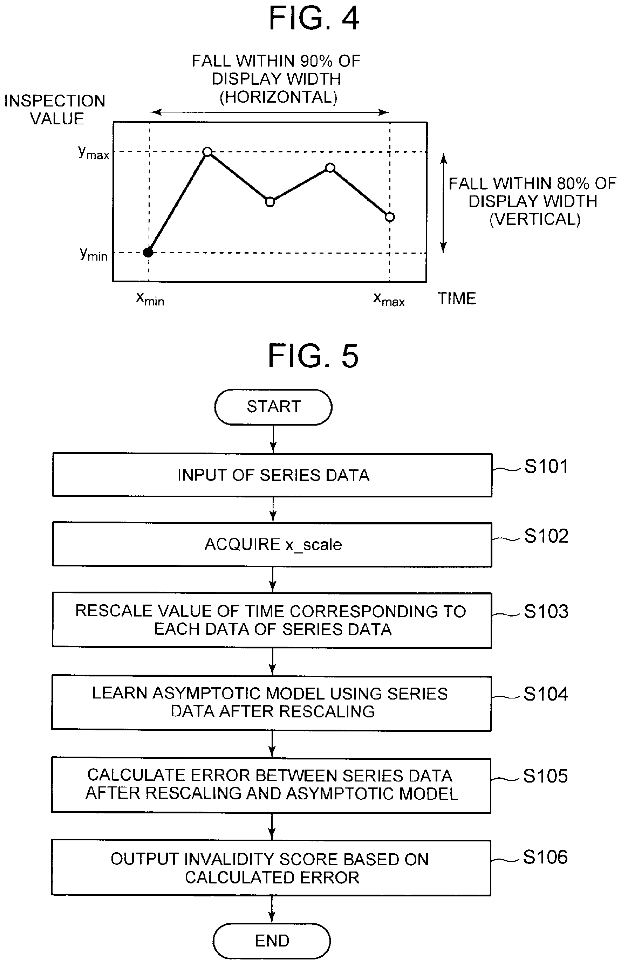

Further, Fig. 4 is an explanatory diagram showing an outline of a method of calculating x_scale shown in formula (4). For example, when the aspect ratio is 1 : 2 (Ar = 2) and five points are displayed in the frame, x_scale is calculated as follows.

$$x\_scale = ((y_{max}-y_{min}) / 0.8*2*0.9) /4)$$

In the above example, assuming that the unit of time that is associated with each data included in the series data is the prediction unit (that is, the number that increases by 1 each time the prediction time point increases by 1), x_scale is calculated as an index representing the x-direction interval of each data expressed in the unit of the y-axis. Therefore, when the unit of time in the series data is other than the prediction unit time, or when the unit of the x direction interval when displaying is other than 1, the x coordinate associated with each data may be divided by the prediction unit time or the unit of the x-direction interval to set the unit of the x axis to 1, and then multiplied by x_scale.

The score calculation unit 11 can also accept the designation of x_scale. For example, the score calculation unit 11 may input x_scale together with the series data. Note that the score calculation unit 11 can also calculate the x_scale by inputting the above-mentioned display parameter together with the series data.

Next, the operation of the present exemplary embodiment will be described. Fig. 5 is a flowchart showing an example of an operation of the index computation device of the first exemplary embodiment.

In the example shown in Fig. 5, first, the score calculation unit 11 inputs the series data to be evaluated (step S101).

Next, the score calculation unit 11 acquires the rescale parameter x_scale (step S102). The x_scale may be input together with the series data, or may be calculated based on the display parameter as described above.

Next, the score calculation unit 11 rescales the value (x coordinate) of time associated with each data included in the series data, based on the acquired rescale parameter x_scale (step S103).

Next, the score calculation unit 11 uses the rescaled series data to learn the asymptotic model (step S104).

Next, the score calculation unit 11 calculates the error between the rescaled series data and the learned asymptotic model (step S105). Here, as the error, the sum of outputs of the error function loss() at each designated time point in the series data is obtained.

Finally, the score calculation unit 11 outputs an invalidity score based on the calculated error (step S106). The score calculation unit 11 may directly output the error as an invalidity score, or may calculate, for example, the average (time point average) at each time point or the average (interval average) at a predetermined section (e.g., three time point section) from the calculated error and output it as an invalidity score.

As described above, according to the present exemplary embodiment, it is possible to output an invalidity score that is an objective index that can determine the invalidity of the input series data without using the actual value. Therefore, by using the invalidity score output according to the present exemplary embodiment, the invalidity of the series data including the predicted value obtained based on the assumed conditions can be automatically determined.

Second exemplary embodiment

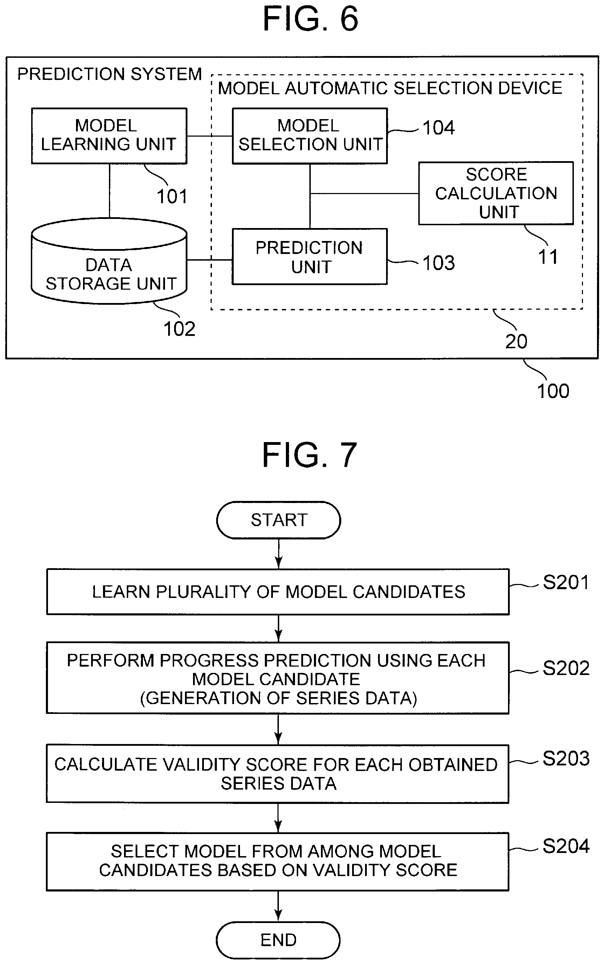

Next, a second exemplary embodiment will be described. In the present exemplary embodiment, a prediction system having a model selection function will be described as one of usage examples of the index computation device 10 of the first exemplary embodiment. Fig. 6 is a block diagram showing a configuration example of the prediction system of the present exemplary embodiment. A prediction system 100 shown in Fig. 6 includes a model learning unit 101, a data storage unit 102, a prediction unit 103, a model selection unit 104, and a score calculation unit 11.

Note that, the example shown in Fig. 6 is an example in which a model automatic selection device 20 including the model selection unit 104, the prediction unit 103, and the score calculation unit 11 is newly added to the prediction system 100 including the model learning unit 101 and the data storage unit 102. However, in the prediction system 100, the model selection unit 104, the prediction unit 103, and the score calculation unit 11 are not necessarily limited to the configuration implemented in one device.

The model learning unit 101 learns a plurality of model candidates that are candidates for a prediction model that predicts the value of a predetermined prediction target item, such as the value of a certain inspection item. The model learning unit 101 may, for example, learn a plurality of model candidates having different combinations of explanatory variables, constraint conditions, and various model parameters as candidates for a prediction model that predicts the value of the designated prediction target item.

The data storage unit 102 stores learning data used for model learning in the model learning unit 101 and information on prediction model candidates learned in the model learning unit 101.

Further, the data storage unit 102 stores prediction data that is data to be input to each model candidate in order to obtain a predicted value from each of the plurality of learned model candidates, and is a data set of explanatory variables used for each model candidate. Note that the application or the like of the predicted value obtained from the prediction data is not particularly limited. For example, the prediction data may be verification data for verifying the prediction model candidates, or may be prediction target data (for example, a data set of explanatory variables including past inspection values and values of one or more inquiry items related to lifestyle habits of an actual user) for obtaining predicted values actually used during the operation of the prediction system, such as future inspection values of the actual user.

The prediction unit 103 performs progress prediction using each of the plurality of learned model candidates and the prediction data stored in the data storage unit 102, and generates series data of prediction target items for each model candidate.

Here, the progress prediction means obtaining a predicted value at each prediction time point included in an evaluation target period, the evaluation target period being a period including two or more time points in a predetermined prediction time unit from a predetermined reference point which is a time point having at least an actual value, and a period in which at least one of the time points is a prediction time point. Note that the prediction time unit may be a standard time interval capable of outputting a predicted value set in advance in a prediction model or a prediction model candidate, such as "one year" if the prediction model is to obtain a predicted value every year.

For example, the prediction unit 103 applies the prediction data stored in the data storage unit 102 to each of the plurality of learned model candidates to obtain a predicted value at each prediction time point included in the predetermined evaluation target period. Then, the prediction unit 103 may generate, for each model candidate, series data including three or more pieces of data indicating the value of the prediction target item in association with time together with data indicating the obtained predicted value.

The prediction unit 103, when performing the progress prediction, applies the prediction data to each model candidate under the condition that some values of the explanatory variables included in the prediction data are made constant to obtain a predicted value at each prediction time point, and generates series data including the obtained predicted value. The prediction unit 103, for example, may apply the prediction data with the items related to lifestyle habits made constant to each of the model candidates for predicting a predetermined inspection value to obtain a predicted value (inspection value) at each prediction time point as a prediction result. In that case, series data including the obtained predicted value (inspection value) at each prediction time point is generated. The series data may include an actual measurement value used for prediction.

The score calculation unit 11 receives, as an input, the series data acquired as the prediction result for each prediction model candidate by the prediction unit 103, and calculates invalidity score for each piece of series data.

The model selection unit 104 selects a prediction model that obtains a predicted value from among a plurality of prediction model candidates, based on the invalidity score for each piece of series data calculated by the score calculation unit 11. The model selection unit 104 may select, for example, a model having the lowest invalidity score. Note that the number of prediction models selected by the model selection unit 104 is not limited to one, and, for example, the model selection unit 104 may select, for example, a predetermined number of prediction models from the one having the lowest invalidity score, or can select all models each having the invalidity score equal to or less than a predetermined threshold.

Further, the model selection unit 104, when having obtained a plurality of pieces of series data (for example, series data corresponding to a plurality of prediction samples) from one prediction model (in this case, a prediction model candidate), can also select a model by combining the invalidity scores for the plurality of pieces of series data. In that case, the model selection unit 104 may select a model by the following method, for example.

(1) Count the number of samples (series data) each having the invalidity score larger than the given threshold as the number of defective samples for each model, and select a predetermined number of (one or more) models in ascending order of the number of defective samples.

(2) Calculate the sum of the invalidity scores for a plurality of pieces of series data for each model, and select a predetermined number of models are in ascending order of the sum.

(3) Calculate the maximum value of the invalidity score for a plurality of pieces of series data for each model, and select a predetermined number of models in ascending order of the maximum value.

Further, the model selection unit 104, when selecting the prediction model, can also select a prediction model that obtains a predicted value from among a plurality of prediction model candidates based on the invalidity score of each piece of series data, and the prediction accuracy of the prediction model (in this example, a plurality of prediction model candidates) that has generated the predicted value included in each piece of series data. By not only evaluating the graph shape (invalidity score) but also evaluating the prediction accuracy, it is possible to select a model with a good balance between the prediction accuracy and the number of defective samples.

The model selection unit 104 may apply, for example, predetermined verification data (for example, a data set consisting of a combination of explanatory variables with the known value of the target variable) to each of the prediction models to be evaluated to calculate a prediction accuracy (for example, root mean square error (RMSE) or correlation coefficient) based on the difference between the obtained predicted value and the target value. Then, the model selection unit 104 may perform model selection based on the invalidity score, for example, from among the prediction models whose prediction accuracy is equal to or higher than a predetermined threshold.

Fig. 7 depicts a flowchart illustrating an operation example of the prediction system of the present exemplary embodiment. In the example shown in Fig. 7, first, the model learning unit 101 learns a plurality of model candidates for one prediction target item (step S201).

Then, the prediction unit 103 performs progress prediction on each of the plurality of model candidates learned by the model learning unit 101, and generates series data including the obtained predicted value for each model candidate (step S202).

Next, the score calculation unit 11 calculates an invalidity score for the series data for each model candidate obtained by the prediction unit 103 (step S203).

Finally, the model selection unit 104 selects a prediction model that obtains a predicted value from among a plurality of model candidates based on the invalidity score calculated by the score calculation unit 11 (step S204).

As described above, according to the present exemplary embodiment, from among several prediction model candidates, it is possible to automatically select a model that outputs a more valid progress prediction, or exclude a model that outputs an invalid progress prediction.

The above example shows an example in which the model selection unit 103 selects at least one model (prediction model candidate), but the model selection unit 103, for example, can also determine, for a plurality of prediction model candidates, availability of shipment based on the invalidity score of the series data including the predicted values obtained from the plurality of prediction model candidates. In that case, for example, the model selection unit 103 may perform a threshold determination on the invalidity score of the series data including the predicted value obtained from each of the plurality of prediction model candidates, and if it is less than or equal to the predetermined threshold, may determine that shipment is OK, and otherwise, may determine that shipment is NG. Further, the model selection unit 103, for example, when having obtained a plurality of pieces of series data from one model, may perform a threshold determination on the number of defective samples, or the sum or maximum value of the invalidity scores for each model, and then, if it is less than or equal to the predetermined threshold, may determine that the shipment is OK, and otherwise, may determine that the shipment is NG. Also in this example, it is possible to perform shipping determination based on the invalidity score and the prediction accuracy. In that case, for example, the model selection unit 103 may determine that the shipment is OK when the prediction accuracy is equal to or higher than a predetermined threshold and the invalidity score satisfies the above condition, and otherwise may determine that the shipment is NG.

As a result of the shipping determination, the model selection unit 103, if the shipment is OK, may ship the model (prediction model candidate) (output to the outside), and if the shipment is NG, may perform predetermined alert processing.

The alert processing may include, for example, outputting the effect to a predetermined server or a display device, together with an identifier of a model for which the shipment is determined to be NG, the series data at that time, its invalidity score, and the like, and requesting manual shipping availability determination. Further, the result of manual shipment availability determination may be accepted.

Third exemplary embodiment

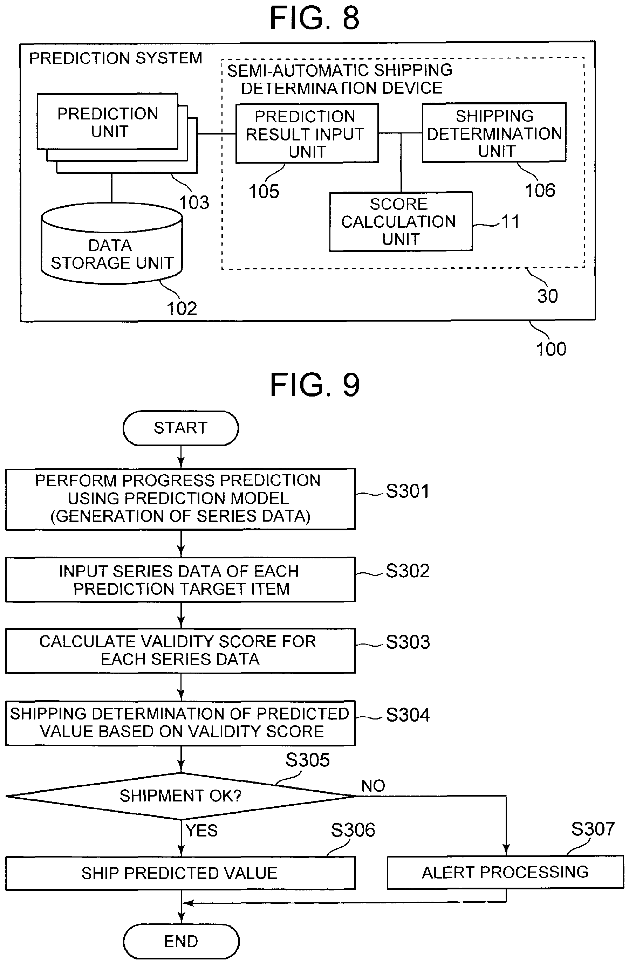

Next, a third exemplary embodiment will be described. In the present exemplary embodiment, a prediction system having a shipping determination function will be described as one of usage examples of the index computation device 10 of the first exemplary embodiment. Fig. 8 is a block diagram showing a configuration example of the prediction system of the present exemplary embodiment. The prediction system 100 shown in Fig. 8 includes a data storage unit 102, one or more prediction units 103, a prediction result input unit 105, a shipping determination unit 106, and a score calculation unit 11.

Note that, the example shown in Fig. 8 is an example in which a semi-automatic shipping determination device 30 including a prediction result input unit 105, a shipping determination unit 106, and a score calculation unit 11 is newly added to the prediction system 100 including the data storage unit 102 and the prediction unit 103. However, in the prediction system 100, the prediction result input unit 105, the shipping determination unit 106, and the score calculation unit 11 are not necessarily limited to the configuration implemented in one device.

In the present exemplary embodiment, the data storage unit 102 stores information on the learned prediction model corresponding to one or more prediction target items. Further, the data storage unit 102 stores prediction data (prediction sample) that is data to be input to each prediction model to obtain a predicted value from the learned prediction model, and is a data set of explanatory variables used in each prediction model. Note that the prediction data is not limited to one, and may be multiple. The data storage unit 102 may store, for example, a plurality of pieces of prediction data corresponding to each of the designated or predetermined one or more prediction target persons.

Each of the prediction units 103 is associated with one prediction target item, performs progress prediction using the learned prediction model and prediction data of the corresponding prediction target item stored in the data storage unit 102, and generates series data of the corresponding prediction target item.

For example, each of the prediction units 103 reads the learned prediction model of the corresponding prediction target item stored in the data storage unit 102, and applies the prediction data of the corresponding prediction target item stored in the data storage unit 102 to the prediction model to obtain the predicted value at each prediction time point included in the predetermined evaluation target period for the corresponding prediction target item. Then, each of the prediction units 103 may generate series data including three or more pieces of data indicating the value of the prediction target item in association with time, together with the data indicating the obtained predicted value. Note that each of the prediction units 103, when a plurality of pieces of prediction data are stored, may generate series data including data indicating a predicted value obtained by applying the prediction model for each prediction data.

Each of the prediction units 103, similarly to the second exemplary embodiment, when performing the progress prediction, applies the prediction data to the prediction model under the condition that the values of some of the explanatory variables included in the prediction data are made constant to obtain a predicted value at each prediction time point, and generates series data including the obtained predicted value. Each of the prediction units 103, for example, may apply the prediction data with the items related to lifestyle habits made constant to each of the model candidates for predicting the corresponding predetermined inspection value, to obtain the predicted value (the above inspection value) at each prediction time point as a prediction result. In that case, series data including the obtained predicted value (inspection value) at each prediction time point is generated. The series data may include an actual measurement value used for prediction.

The prediction result input unit 105 inputs the series data of each prediction target item obtained from each of the prediction units 103.

The score calculation unit 11 calculates an invalidity score for the series data of each prediction target item input from the prediction result input unit 105.

The shipping determination unit 106 performs shipping availability determination of a predicted value, based on the invalidity score with respect to the series data of each prediction target item calculated by the score calculation unit 11. The shipping determination unit 106, for example, when having obtained a plurality of pieces of series data (for example, series data corresponding to a plurality of prediction samples) from one prediction model, if the invalidity score of all pieces of the series data is equal to or less than a predetermined threshold, may determine that the shipment of the predicted value included in each piece of series data is OK, and otherwise, may perform predetermined alert processing. Further, the shipping determination unit 106, for example, when having obtained the series data from each of the plurality of prediction target items, may evaluate them individually, or can also evaluate them collectively (collective evaluation). As an example, the shipping determination unit 106 may collectively evaluate the series data including the predicted value in the shipping unit for each shipping unit (for example, the prediction target person) which is a unit in which the predicted value is shipped.

The alert processing may include, for example, outputting the effect to a predetermined server or a display device together with an identifier of a model for which the shipment is determined to be NG, the series data at that time, its invalidity score, and the like, and requesting manual shipping availability determination. Further, the result of manual shipment availability determination may be accepted.

In addition, the shipping determination unit 106 may output the predicted value to the outside if the shipment is OK as a result of the shipping availability determination thus obtained finally.

Also in the present exemplary embodiment, the shipping determination unit 106, when performing the shipping determination of the predicted value, can also perform shipping availability determination of the predicted value, based on not only the invalidity score of each piece of series data but also the prediction accuracy of the prediction model that has generated the predicted value. By not only evaluating the graph shape (invalidity score) but also evaluating the prediction accuracy, it is possible to ship a predicted value with a good balance between the prediction accuracy and the number of defective samples. The method of calculating the prediction accuracy may be the same as in the second exemplary embodiment.

Fig. 9 depicts a flowchart illustrating an operation example of the prediction system of the present exemplary embodiment. In the example shown in Fig. 9, first, each of the prediction units 103 performs progress prediction using a learned prediction model for the corresponding prediction target item, and generates series data including the obtained predicted value (step S301).

Then, the prediction result input unit 105 inputs the series data including the prediction result of the prediction unit 103 corresponding to each prediction target item (step S302).

Next, the score calculation unit 11 calculates an invalidity score for each piece of input series data (step S303).

Next, the shipping determination unit 106 performs shipping determination of the obtained predicted value based on the invalidity score of each piece of series data (step S304). Here, the shipping determination unit 106 primarily determines availability of shipment depending on whether or not the invalidity score of all pieces of series data is equal to or less than a predetermined threshold.

As a result of the shipping determination, if the shipment is OK (Yes in step S305), the obtained predicted value is shipped (output to the outside) (step S306).

As a result of the shipping determination, if the shipment is not OK (No in step S305), predetermined alert processing is performed (step S307).

As described above, according to the present exemplary embodiment, in determining whether to output the prediction result to the outside (shipping determination), it is not necessary to perform a visual check by a domain expert each time, so cost and time for shipping can be reduced.

Note that, the above example shows an example in which the shipping determination unit 106 determines availability of shipment of the predicted value, but, as in the second exemplary embodiment, the shipping determination unit 106 can also determine availability of shipment of the prediction model. In that case, the shipping determination unit 106 may determine availability of shipment of the prediction model, for example, based on the invalidity score calculated for one or more pieces of series data including a predicted value obtained by applying predetermined verification data to the prediction model to be determined. Note that, also in this example, the shipping determination unit 106 may determine availability of shipment of the prediction model, based on not only the invalidity score but also the prediction accuracy of the prediction model.

Further, the shipping determination unit 106 is similar to the above in that, for example, when the predicted values for a plurality of samples are obtained from one model, the shipping determination unit 106 performs shipping determination by combining the series data including the predicted values in each sample. Note that, for example, when the series data is obtained from each of a plurality of prediction target items, the shipping determination unit 106 may evaluate them individually, or can also evaluate them collectively (collective evaluation). Further, the shipping determination unit 106 may collectively evaluate, for each shipping unit that is a unit in which the predicted value or the prediction model is shipped, the series data including the predicted value in the shipping unit.

In addition, each of the above-described exemplary embodiments exemplifies a method of evaluating the invalidity of the series data of the inspection value including the predicted value of the inspection item when the items related to lifestyle habits are made constant, but the prediction target and assumed conditions are not limited to these.

Further, in the above, the asymptotic model is illustrated as the model to be compared, but the model to be compared may be other than the asymptotic model. That is, when the valid function form is preliminarily determined for the input series data, the fitting to the valid function form is performed by the same method to obtain the curve model to be compared, and thereby similar effect can be obtained.

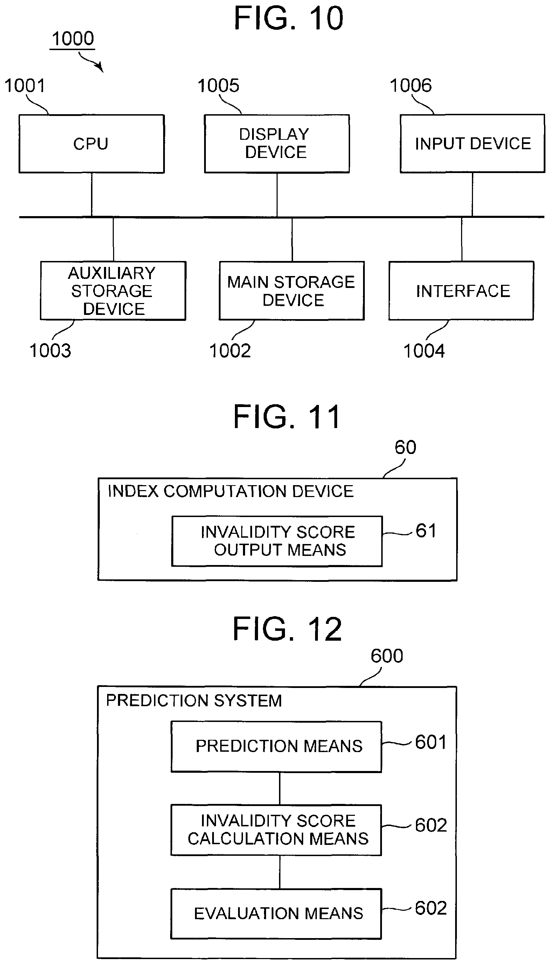

Further, Fig. 10 is a schematic block diagram showing a configuration example of a computer according to the exemplary embodiment of the present invention. A computer 1000 includes a CPU 1001, a main storage device 1002, an auxiliary storage device 1003, an interface 1004, a display device 1005, and an input device 1006.

The system, the server, and other devices in the above-described exemplary embodiments may be installed in the computer 1000. In that case, the operation of each device may be stored in the auxiliary storage device 1003 in the form of a program. The CPU 1001 reads the program from the auxiliary storage device 1003, expands it in the main storage device 1002, and executes the predetermined processing in each exemplary embodiment according to the program. The CPU 1001 is an example of an information processing device that operates according to a program, and may include, in addition to the CPU (Central Processing Unit), for example, MPU (Micro Processing Unit), MCU (Memory Control Unit), and GPU (Graphics Processing Unit), etc.

The auxiliary storage device 1003 is an example of a non-transitory tangible medium. Other examples of non-transitory tangible medium include a magnetic disk, a magneto-optical disk, CD-ROM, DVD-ROM, a semiconductor memory, or the like that is connected via the interface 1004. When the program is distributed to the computer 1000 through a communication line, the computer 1000 having received the distribution may expand the program into the main storage device 1002 and execute predetermined processing in each exemplary embodiment.

Further, the program may be a program for realizing a part of the predetermined processing in the above exemplary embodiment. Further, the program may be a difference program that realizes predetermined processing in each exemplary embodiment in combination with another program already stored in the auxiliary storage device 1003.

The interface 1004 transmits/receives information to/from other devices. The display device 1005 also presents information to the user. Further, the input device 1006 accepts input of information from the user.

Further, depending on the processing content in the exemplary embodiment, some elements of the computer 1000 can be omitted. For example, the display device 1005 can be omitted if the computer 1000 does not present information to the user. For example, if the computer 1000 does not accept information input from the user, the input device 1006 can be omitted.

Also, some or all of the components of the above-described exemplary embodiments may be implemented by a general-purpose or dedicated circuit (circuitry), a processor or the like, or a combination thereof. These may be configured by a single chip or may be configured by a plurality of chips connected via a bus. Some or all of the components of the above-described exemplary embodiments may be realized by a combination of the above-described circuitry and the like and a program.

When some or all of the components of the above-described exemplary embodiments are realized by a plurality of information processing devices or circuits, the plurality of information processing devices or circuits may be centrally arranged or distributed. For example, the information processing device, the circuit, and the like may be realized as a form in which a client and server system, a cloud computing system, and the like are connected to each other via a communication network.

Next, summary of the present invention will be described. Fig. 11 is a block diagram showing an outline of the index computation device of the present invention. An index computation device 60 shown in Fig. 11 includes invalidity score output means 61.

The invalidity score output means 61 (e.g., the score calculation unit 11) outputs, when series data of a predetermined prediction target item is input, the series data including three or more pieces of data which indicate the value of the prediction target item in association with time, and at least one of which is data indicating a predicted value, an invalidity score that is an index indicating the invalidity of the series data and is based on an error between a curve model obtained by fitting the series data to a predetermined function form and the series data.

With the above configuration, it is possible to output an invalidity score, which is an objective index for determining the invalidity of input series data, without using the actual value. Therefore, the invalidity of the series data including the predicted value obtained based on the assumed conditions can be automatically determined.

Fig. 12 is a block diagram showing the outline of the prediction system of the present invention. A prediction system 600 shown in Fig. 12 includes prediction means 601, invalidity score calculation means 602, and evaluation means 603. The invalidity score calculation means 602 may be realized by the invalidity score output means 61 included in the index computation device 60.

The prediction means 601 (for example, the prediction unit 103) obtains a predicted value at a predetermined prediction time point using a learned prediction model for a predetermined prediction target item, and also generates data series data including the obtained predicted value, the data series data including three or more pieces of data which indicate the value of the prediction target item in association with time, and at least one of which indicates a predicted value.

The invalidity score calculation means 602 (for example, the score calculation unit 11) calculates an invalidity score that is an index indicating the invalidity of the series data and is based on an error between a curve model obtained by fitting the series data to a predetermined function form and the series data.

The evaluation means 603 (for example, the model selection unit 104 or the shipping determination unit 106), evaluates the series data, a predicted value included in the series data, or a prediction model that has obtained the predicted value, based on the invalidity score calculated by the invalidity score calculation means 602.

With such a configuration, it is possible to determine whether the series data including the predicted value obtained by the prediction means 601, the predicted value included in the series data, and the prediction model that has obtained the predicted value are valid or not, automatically select a more valid one from among some candidates, or exclude an invalid one.

The above exemplary embodiment can be described as the following supplementary notes.

(Supplementary note 1) An index computation device, including invalidity score output means that outputs, when series data of a predetermined prediction target item is input, the series data including three or more pieces of data which indicate the value of the prediction target item in association with time, and at least one of which is data indicating a predicted value, an invalidity score that is an index indicating the invalidity of the series data and is based on an error between a curve model obtained by fitting the series data to a predetermined function form and the series data.

(Supplementary note 2) The index computation device according to supplementary note 1, in which the predetermined function form is a predetermined function form that satisfies a condition that an output value converges to a certain value when time is infinite.

(Supplementary note 3) The index computation device according to supplementary note 1 or 2, in which the invalidity score output means performs fitting to the predetermined function form using data belonging to a predetermined first group of the series data, calculates the error with the curve model obtained by the fitting using data belonging to a predetermined second group of the series data, and outputs the invalidity score based on the calculated error, and the data belonging to the first group and the data belonging to the second group do not completely match.

(Supplementary note 4) The index computation device according to supplementary note 3, in which part of the data included in the series data is the first group, and data that does not belong to the first group or all the data of the series data is the second group.

(Supplementary note 5) The index computation device according to any one of supplementary notes 1 to 4, in which the invalidity score output means, before performing the fitting to the predetermined function form, converts a value of the time associated with each data included in the series data, in accordance with a display scale of the series data.

(Supplementary note 6) A prediction system, including: prediction means that obtains a predicted value at a predetermined prediction time point by using a learned prediction model for a predetermined prediction target item, and generates series data including the obtained predicted value and including three or more pieces of data which indicate the value of the prediction target item in association with time, and at least one of which is data indicating a predicted value; invalidity score calculation means that calculates an invalidity score that is an index indicating the invalidity of the series data and is based on an error between a curve model obtained by fitting the series data to a predetermined function form and the series data; and evaluation means that performs evaluation based on the invalidity score on the series data, a predicted value included in the series data, or a prediction model that has obtained the predicted value.

(Supplementary note 7) The prediction system according to supplementary note 6, in which the evaluation means performs evaluation on the series data, the predicted value included in the series data, or the prediction model that has obtained the predicted value, based on the invalidity score and a prediction accuracy of the prediction model calculated by using predetermined verification data.

(Supplementary note 8) The prediction system according to supplementary note 6 or 7, further including model learning means that learns a plurality of model candidates for the predetermined prediction target item, in which the prediction means obtains the predicted value using each of the plurality of model candidates, and generates series data including the obtained predicted value, for each of the plurality of model candidates, the invalidity score calculation means calculates the invalidity score for the series data for each of the plurality of model candidates, and the evaluation means performs the evaluation on the plurality of model candidates, and selects a model that obtains a predicted value of the prediction target item from among the plurality of model candidates based on the evaluation result.

(Supplementary note 9) The prediction system according to supplementary note 6 or 7, in which the one or more prediction means are provided corresponding to each of the one or more prediction target items, the invalidity score output means calculates the invalidity score for the series data for each of the prediction target items obtained from the one or more prediction means, and the evaluation means performs the evaluation on the series data for each of the prediction target items, and performs shipping determination of the predicted value based on the evaluation result.

(Supplementary note 10) A progress prediction evaluation method including: when series data of a predetermined prediction target item is input, the series data including three or more pieces of data which indicate the value of the prediction target item in association with time, and at least one of which is data indicating a predicted value, calculating, by an information processing device, an invalidity score that is an index indicating the invalidity of the series data and is based on an error between a curve model obtained by fitting the series data to a predetermined function form and the series data; and evaluating, by the information processing device, the series data, a predicted value included in the series data, or a prediction model that has obtained the predicted value, based on the invalidity score.

(Supplementary note 11) A progress prediction evaluation program causing a computer to execute the processes of, when series data of a predetermined prediction target item is input, the series data including three or more pieces of data which indicate the value of the prediction target item in association with time, and at least one of which is data indicating a predicted value, calculating an invalidity score that is an index indicating the invalidity of the series data and is based on an error between a curve model obtained by fitting the series data to a predetermined function form and the series data; and evaluating the series data, a predicted value included in the series data, or a prediction model that has obtained the predicted value, based on the invalidity score.

Although the present invention has been described above with reference to the exemplary embodiments and examples, the present invention is not limited to the above-described exemplary embodiments and examples. Various modifications that can be understood by those skilled in the art can be made to the configuration and details of the present invention within the scope of the present invention.

This application claims the priority on the basis of Japanese patent application 2018-067622 for which it applied on March 30, 2018, and takes in its entirety of the disclosure herein.

Industrial Applicability

The present invention provides not only series data including a predicted value obtained based on assumed conditions, but also a valid function form for series data including three or more data indicating the value of a prediction target item in association with time. If it is possible, it is suitably applicable.

Reference Signs List

| | |
|---|---|
| 10 | Index computation device |
| 11 | Score calculation unit |
| 20 | Model automatic selection device |
| 30 | Semi-automatic shipping determination device |
| 100 | Prediction system |
| 101 | Model learning unit |
| 102 | Data storage unit |
| 103 | Prediction unit |
| 104 | Model selection unit |
| 105 | Prediction result input unit |
| 106 | Shipping determination unit |
| 1000 | Computer |
| 1001 | CPU |
| 1002 | Main storage device |
| 1003 | Auxiliary storage device |
| 1004 | Interface |
| 1005 | Display device |
| 1006 | Input device |
| 60 | Index computation device |
| 61 | Invalidity score output means |
| 600 | Prediction system |
| 601 | Prediction means |
| 602 | Invalidity score calculation means |
| 603 | Evaluation means |

**Claims**

1. An index computation device, comprising
   invalidity score output means that outputs, when series data of a predetermined prediction target item is input, the series data including three or more pieces of data which indicate the value of the prediction target item in association with time, and at least one of which is data indicating a predicted value, an invalidity score that is an index indicating the invalidity of the series data and is based on an error between a curve model obtained by fitting the series data to a predetermined function form and the series data.

2. The index computation device according to claim 1, wherein
   the predetermined function form is a predetermined function form that satisfies a condition that an output value converges to a certain value when time is infinite.

3. The index computation device according to claim 1 or 2, wherein
   the invalidity score output means performs fitting to the predetermined function form using data belonging to a predetermined first group of the series data, calculates an error with the curve model obtained by the fitting using data belonging to a predetermined second group of the series data, and outputs the invalidity score based on the calculated error, and
   the data belonging to the first group and the data belonging to the second group do not completely match.

4. The index computation device according to claim 3, wherein
   part of the data included in the series data is the first group, and data that does not belong to the first group or all the data of the series data is the second group.

5. The index computation device according to any one of claims 1 to 4, wherein
the invalidity score output means, before performing the fitting to the predetermined function form, converts a value of the time associated with each data included in the series data, in accordance with a display scale of the series data.

6. A prediction system, comprising:

prediction means that obtains a predicted value at a predetermined prediction time point by using a learned prediction model for a predetermined prediction target item, and generates series data including the obtained predicted value and including three or more pieces of data which indicate the value of the prediction target item in association with time, and at least one of which is data indicating a predicted value;
invalidity score calculation means that calculates an invalidity score that is an index indicating the invalidity of the series data and is based on an error between a curve model obtained by fitting the series data to a predetermined function form and the series data; and
evaluation means that performs evaluation based on the invalidity score on the series data, a predicted value included in the series data, or a prediction model that has obtained the predicted value.

7. The prediction system according to claim 6, wherein
the evaluation means performs evaluation on the series data, the predicted value included in the series data, or the prediction model that has obtained the predicted value, based on the invalidity score and a prediction accuracy of the prediction model calculated by using predetermined verification data.

8. The prediction system according to claim 6 or 7, further comprising
model learning means that learns a plurality of model candidates for the predetermined prediction target item, wherein the prediction means obtains the predicted value using each of the plurality of model candidates, and generates series data including the obtained predicted value, for each of the plurality of model candidates,
the invalidity score calculation means calculates the invalidity score for the series data for each of the plurality of model candidates, and
the evaluation means performs the evaluation on the plurality of model candidates, and selects a model that obtains a predicted value of the prediction target item from among the plurality of model candidates based on the evaluation result.

9. The prediction system according to claim 6 or 7, wherein
the one or more prediction means are provided corresponding to each of the one or more prediction target items,
the invalidity score output means calculates the invalidity score for the series data for each of the prediction target items obtained from the one or more prediction means, and
the evaluation means performs the evaluation on the series data for each of the prediction target items, and performs shipping determination of the predicted value based on the evaluation result.

10. A progress prediction evaluation method comprising:

when series data of a predetermined prediction target item is input, the series data including three or more pieces of data which indicate the value of the prediction target item in association with time, and at least one of which is data indicating a predicted value,
calculating, by an information processing device, an invalidity score that is an index indicating the invalidity of the series data and is based on an error between a curve model obtained by fitting the series data to a predetermined function form and the series data; and
evaluating, by the information processing device, the series data, a predicted value included in the series data, or a prediction model that has obtained the predicted value, based on the invalidity score.

11. A progress prediction evaluation program causing a computer to execute the processes of,
when series data of a predetermined prediction target item is input, the series data including three or more pieces of data which indicate the value of the prediction target item in association with time, and at least one of which is data indicating a predicted value,
calculating an invalidity score that is an index indicating the invalidity of the series data and is based on an error between a curve model obtained by fitting the series data to a predetermined function form and the series data; and
evaluating the series data, a predicted value included in the series data, or a prediction model that has obtained the predicted value, based on the invalidity score.

# FIG. 1

INDEX COMPUTATION DEVICE

SERIES DATA —— SCORE CALCULATION UNIT —— SCORE

11

10

# FIG. 2A

INSPECTION VALUE y

PREDICTION TIME POINT 1

PREDICTION TIME POINT 2

PREDICTION TIME POINT 3

ASYMPTOTIC MODEL CURVE

NORMAL RANGE

SCORE SMALL

0    1    2    3    TIME x

# FIG. 2B

INSPECTION VALUE y

PREDICTION TIME POINT 1

PREDICTION TIME POINT 2

ASYMPTOTIC MODEL CURVE

NORMAL RANGE

SCORE LARGE

PREDICTION TIME POINT 3

0    1    2    3    TIME x

# FIG. 3A

INSPECTION VALUE

D={(0, 140),
(1, 150),
(2, 160),
(3, 120)}

# FIG. 3B

INSPECTION VALUE

D={(0, 140),
(1, 150),
(2, 160),
(3, 120)}

# FIG. 3C

INSPECTION VALUE

D'={(   0, 140),
(  50, 150),
(100, 160),
(150, 120)}

# FIG. 4

INSPECTION
VALUE

FALL WITHIN 90% OF
DISPLAY WIDTH
(HORIZONTAL)

$y_{max}$

$y_{min}$

$x_{min}$           $x_{max}$           TIME

FALL WITHIN 80% OF
DISPLAY WIDTH
(VERTICAL)

# FIG. 5

START

INPUT OF SERIES DATA — S101

ACQUIRE x_scale — S102

RESCALE VALUE OF TIME CORRESPONDING TO
EACH DATA OF SERIES DATA — S103

LEARN ASYMPTOTIC MODEL USING SERIES
DATA AFTER RESCALING — S104

CALCULATE ERROR BETWEEN SERIES DATA
AFTER RESCALING AND ASYMPTOTIC MODEL — S105

OUTPUT INVALIDITY SCORE BASED ON
CALCULATED ERROR — S106

END

# FIG. 6

PREDICTION SYSTEM

MODEL AUTOMATIC SELECTION DEVICE

MODEL
LEARNING UNIT

MODEL
SELECTION UNIT ~104

101

SCORE
CALCULATION
UNIT

DATA
STORAGE UNIT

PREDICTION
UNIT ~103

11

102

20

100

# FIG. 7

START

LEARN PLURALITY OF MODEL CANDIDATES ~S201

PERFORM PROGRESS PREDICTION USING EACH
MODEL CANDIDATE
(GENERATION OF SERIES DATA) ~S202

CALCULATE VALIDITY SCORE FOR EACH OBTAINED
SERIES DATA ~S203

SELECT MODEL FROM AMONG MODEL
CANDIDATES BASED ON VALIDITY SCORE ~S204

END

# FIG. 8

PREDICTION SYSTEM

SEMI-AUTOMATIC SHIPPING
DETERMINATION DEVICE

PREDICTION
UNIT

PREDICTION
RESULT INPUT
UNIT

SHIPPING
DETERMINATION
UNIT

103

105

106

SCORE
CALCULATION
UNIT

11

DATA
STORAGE UNIT

30

102

100

# FIG. 9

START

PERFORM PROGRESS PREDICTION
USING PREDICTION MODEL
(GENERATION OF SERIES DATA) — S301

INPUT SERIES DATA OF EACH
PREDICTION TARGET ITEM — S302

CALCULATE VALIDITY SCORE FOR
EACH SERIES DATA — S303

SHIPPING DETERMINATION OF PREDICTED
VALUE BASED ON VALIDITY SCORE — S304

S305

SHIPMENT OK?   NO

YES   S306   S307

SHIP PREDICTED VALUE   ALERT PROCESSING

END

# FIG. 10

<u>1000</u>

1001          1005          1006

| CPU | DISPLAY DEVICE | INPUT DEVICE |

| AUXILIARY STORAGE DEVICE | MAIN STORAGE DEVICE | INTERFACE |

1003          1002          1004

# FIG. 11

60

INDEX COMPUTATION DEVICE

INVALIDITY SCORE OUTPUT MEANS — 61

# FIG. 12

600

PREDICTION SYSTEM

PREDICTION MEANS — 601

INVALIDITY SCORE CALCULATION MEANS — 602

EVALUATION MEANS — 602

# FIG. 13A

INSPECTION VALUE

THIS YEAR

1 YEAR
LATER

2 YEARS
LATER

3 YEARS
LATER

1 YEAR
BEFORE

TIME

# FIG. 13B

INSPECTION VALUE

THIS YEAR

2 YEARS
LATER    3 YEARS
LATER

1 YEAR
BEFORE

1 YEAR
LATER

TIME

**INTERNATIONAL SEARCH REPORT**

| International application No. |
| --- |
| PCT/JP2018/043910 |

A. CLASSIFICATION OF SUBJECT MATTER
Int.Cl. G06Q10/04(2012.01)i

According to International Patent Classification (IPC) or to both national classification and IPC

B. FIELDS SEARCHED

Minimum documentation searched (classification system followed by classification symbols)
Int.Cl. G06Q10/04

Documentation searched other than minimum documentation to the extent that such documents are included in the fields searched
Published examined utility model applications of Japan     1922-1996
Published unexamined utility model applications of Japan   1971-2019
Registered utility model specifications of Japan           1996-2019
Published registered utility model applications of Japan   1994-2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y<br>A | JP 2011-176984 A (TOSHIBA CORPORATION) 08 September 2011, paragraphs [0069], [0070] (Family: none) | 1-7, 9-11<br>8 |
| Y<br>A | JP 2016-201869 A (LEMONGAS CO., LTD.) 01 December 2016, paragraphs [0063], [0073] (Family: none) | 1-7, 9-11<br>8 |
| Y<br>A | JP 2005-63208 A (NIPPON TELEGRAPH AND TELEPHONE CORPORATION) 10 March 2005, paragraph [0003], fig. 5 (Family: none) | 2-5<br>1, 6-11 |

☒ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>06.02.2019 | Date of mailing of the international search report<br>19.02.2019 |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2018/043910

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | WO 2005/031278 A1 (NOZZLE NETWORK CO., LTD.) 07 April 2005, paragraphs [0079]-[0081]<br>(Family: none) | 5<br>1-4, 6-11 |
| A | JP 2017-49677 A (FUJITSU LIMITED) 09 March 2017, entire text, all drawings<br>& US 2017/0061329 A1, entire text, all drawings | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10228463 A

- JP 2018067622 A

**Non-patent literature cited in the description**

- **HIDETAKA TAKAHASHI ; KATSUMI YOSHIDA.** HRA (Health Risk Appraisal System) for Lifestyle Improvement. *Journal of Japan Society of Human Dock (JHD),* 1997, vol. 11 (4), 123-128